# EUROPEAN PATENT SPECIFICATION

(11) **EP 2 874 695 B1**
(45) Date of publication and mention of the grant of the patent: **14.02.2018**
(21) Application number: 13732731.8
(22) Date of filing: 11.06.2013
(51) Int. Cl.: A61N 1/37, H04L 27/14

(54) **RECEIVER WITH DUAL BAND PASS FILTERS AND DEMODULATION CIRCUITRY FOR AN EXTERNAL CONTROLLER USEABLE IN AN IMPLANTABLE MEDICAL DEVICE SYSTEM**
EMPFÄNGER MIT ZWEI BANDPASSFILTERN UND DEMODULATIONSSCHALTUNG FÜR EINE EXTERNE STEUERUNG ZUR VERWENDUNG IN EINEM SYSTEM MIT IMPLANTIERBAREN MEDIZINISCHEN VORRICHTUNGEN
RÉCEPTEUR À DOUBLES FILTRES PASSE-BANDE ET CIRCUITERIE DE DÉMODULATION POUR UN DISPOSITIF DE COMMANDE EXTERNE UTILISABLE DANS UN SYSTÈME DE DISPOSITIF MÉDICAL IMPLANTABLE

(30) Priority: 20.07.2012 US 201261673820 P; 23.05.2013 US 201313900877
(43) Date of publication of application: 27.05.2015
(73) Proprietor: Boston Scientific Neuromodulation Corporation, Valencia, CA 91355 (US)
(72) Inventor: STOUFFER, Thomas, W., Chatsworth, CA 91311 (US); AGHASSIAN, Daniel, Glendale, CA 91208 (US); FREIDIN, Lev, Simi Valley, CA 93603 (US); DRONOV, Vasily, San Jose, CA 95125 (US)
(74) Representative: Vossius & Partner Patentanwälte Rechtsanwälte mbB
(86) International application number: PCT/US2013/045116
(87) International publication number: WO 2014/014582

(56) References cited:
- US-A- 4 568 882
- US-A- 5 471 187
- US-A1- 2005 131 494
- US-A1- 2011 112 610

## Description

### FIELD OF THE INVENTION

The present invention relates to improved receiver and demodulation circuitry useable in an external controller that communicates with an implantable medical device.

### BACKGROUND

Implantable stimulation devices deliver electrical stimuli to nerves and tissues for the therapy of various biological disorders, such as pacemakers to treat cardiac arrhythmia, defibrillators to treat cardiac fibrillation, cochlear stimulators to treat deafness, retinal stimulators to treat blindness, muscle stimulators to produce coordinated limb movement, spinal cord stimulators to treat chronic pain, cortical and deep brain stimulators to treat motor and psychological disorders, and other neural stimulators to treat urinary incontinence, sleep apnea, shoulder sublaxation, etc. The description that follows will generally focus on the use of the invention within a Spinal Cord Stimulation (SCS) system, such as that disclosed in U.S. Patent 6,516,227. However, the present invention may find applicability in any implantable medical device system.

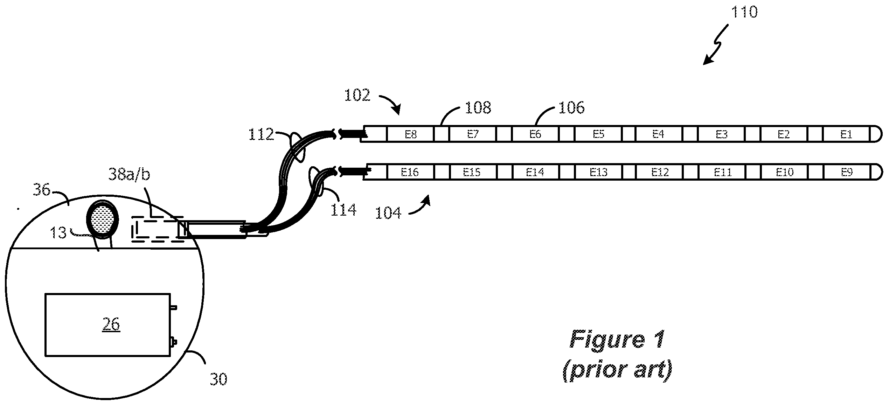

As shown in Figure 1, a SCS system typically includes an Implantable Pulse Generator (IPG) 100, which includes a biocompatible device case 30 formed of titanium for example. The case 30 typically holds the circuitry and battery 26 necessary for the IPG to function, although IPGs can also be powered via external RF energy and without a battery. The IPG 100 is coupled to electrodes 106 via one or more electrode leads (two such leads 102 and 104 are shown), such that the electrodes 106 form an electrode array 110. The electrodes 106 are carried on a flexible body 108, which also houses the individual signal wires 112 and 114 coupled to each electrode. In the illustrated embodiment, there are eight electrodes on lead 102, labeled E₁-E₈, and eight electrodes on lead 104, labeled E₉-E₁₆, although the number of leads and electrodes is application specific and therefore can vary. The leads 102 and 104 couple to the IPG 100 using lead connectors 38a and 38b, which are fixed in a header material 36, which can comprise an epoxy for example. In a SCS application, electrode leads 102 and 104 are typically implanted on the right and left side of the dura within the patient's spinal cord. These leads 102 and 104 are then tunneled through the patient's flesh to a distant location, such as the buttocks, where the IPG 100 is implanted.

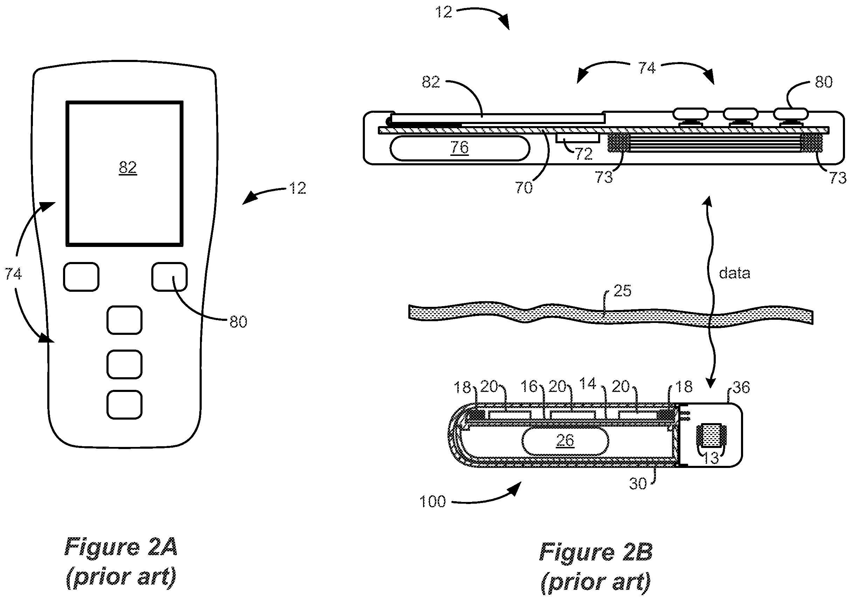

Figure 2A shows a plan view of an external controller 12 used to wirelessly communicate with the IPG 100, while Figure 2B shows a cross section of the external controller 12 and the IPG 100. As shown in Figure 2B, the IPG 100 typically includes an electronic substrate assembly 14 including a printed circuit board (PCB) 16, along with various electronic components 20, such as a microcontroller, integrated circuits, and capacitors mounted to the PCB 16. Two coils are generally present in the IPG 100: a telemetry coil 13 used to transmit/receive data to/from the external controller 12; and a charging coil 18 for charging or recharging the IPG's battery 26 using an external charger (not shown). The telemetry coil 13 can be mounted within the header 36 of the IPG 100 as shown, but can also be provided within the case 30, as disclosed in U.S. Patent Publication 2011/0112610 for example.

The external controller 12, such as a patient hand-held programmer or a clinician's programmer, is used to send data to and receive data from the IPG 100. For example, the external controller 12 can send programming data such as therapy settings to the IPG 100 to dictate the therapy the IPG 100 will provide to the patient. Also, the external controller 12 can act as a receiver of data from the IPG 100, such as various data reporting on the IPG's status. As shown in Figure 2B, the external controller 12, like the IPG 100, also contains a PCB 70 on which electronic components 72 are placed to control operation of the external controller 12. The external controller 12 is powered by a battery 76, but could also be powered by plugging it into a wall outlet for example.

The external controller 12 typically comprises a graphical user interface 74 similar to that used for a portable computer, cell phone, or other hand held electronic device. The graphical user interface 74 typically comprises touchable buttons 80 and a display 82, which allows the patient or clinician to operate the external controller 12 to update the therapy the IPG 100 provides, and to review any relevant status information that has been reported from the IPG 100.

Wireless data transfer between the IPG 100 and the external controller 12 preferably takes place via inductive coupling between a telemetry coil 73 (Fig. 2B) in the external controller 12 and the telemetry coil 13 in the IPG 100. Either coil 13 or 73 can act as the transmitter or the receiver, thus allowing for two-way communication between the two devices. Typically, the transmitting device will send data to the receiving device via a Frequency Shift Keying (FSK) protocol in which different data states are indicated by different frequencies. For example, a transmitting device may send a logic '0' bit to the receiving device at 121 kHz, but may send a logic '1' bit at 129 kHz. That is, the data is represented relative to a center frequency f_{c}=125 kHz, with the logic states representing a +/-4 kHz deviation from this center frequency. Bits may be serially transferred in this fashion at a given rate of 4k bits/sec (4 kHz), i.e., a bit duration of t_{b}=250 µs for example, meaning that a logic '0' bit roughly comprises 30 cycles at 121 kHz (121/4), while a logic '1' bit roughly comprises 32 cycles at 129 kHz (129/4). These frequencies are not significantly attenuated in the patient's tissue 25, and so data transmission can occur transcutaneously using this scheme.

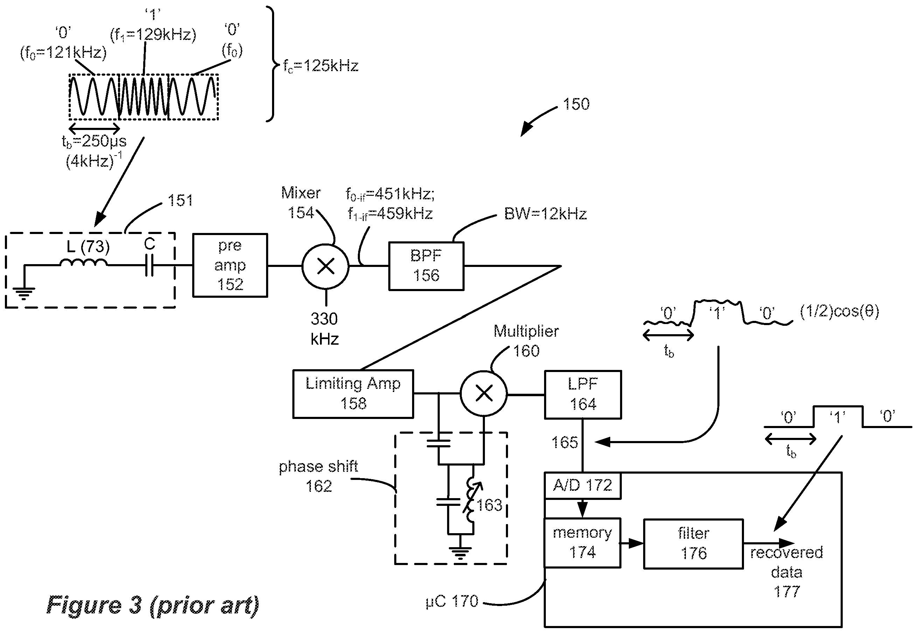

Figure 3 illustrates prior art receiver and demodulation circuitry 150 used in an external controller 12 to receive and recover FSK data transmitted from the IPG 100. The circuitry 150 includes a L-C tank circuit 151 (or antenna, more generally) comprising a serial connection between the telemetry coil 73 and a tank capacitor C. (A parallel arrangement can also be used). The inductance L of the coil 73 or the capacitance of the tank capacitor C can be tuned to allow the tank circuit 151 to generally resonate at the center frequency f_{c}= 125 kHz of the data expected from the IPG 100.

The low-amplitude signal received at coil 73 is amplified at a pre-amplifier 152, where it is them mixed with a 330 kHz reference waveform at a mixer 154 to produce a signal with an intermediate frequency of f_{c-if}=455 kHz. This is done in the prior art because 455 kHz comprises a well-known standard communication frequency, and as a result, receiver components are readily available to operate at this frequency. See, e.g., http:// en.wikipedia.org/wiki/Intermediate_frequency. Mixer 154 can be implemented using Part No. MAX 4636, manufactured by Maxim Integrated Products, Inc.

After mixing, the up-shifted frequency is provided to a band pass filter (BPF) 156, centered at f_{c-if}=455 kHz and with a bandwidth (BW) of 12 kHz. This BPF 156 reduces noise outside of the band of frequencies of interest (i.e., below 449 kHz and above 461 kHz), while allowing the signals from the IPG 100 (f_{0-if}= 121k+330k = 451kHz, and f_{1-if}= 129k+330k = 459kHz) to readily pass. Thereafter, the signals are passed to a limiting amplifier 158 which limits the magnitude of the signals by clipping their peaks if necessary, as is well known. Another BPF similar to BPF 156 can be provided after the limiting amplifier 158 to remove any out-of-band frequency components resulting from clipping, but this is not shown for simplicity. The BFP(s) can comprise ceramic filters, such as Part No. AHCFM2-455AL, manufactured by Toko America, Inc., or Part No. CFUM455D, manufactured by Murata Manufacturing Co.

Thereafter, the received signal is demodulated to recover the transmitted data. This occurs first by sending the signals to a multiplier 160, which multiplies the signal with a phase-shifted version of the signal provided by phase shift block 162. The quad coil 163 in the phase shift block 162 is tunable to provide a 90-degree phase shift at f_{c-if}=455 kHz, but will provide different phase shifts θ for the FSK signals of interest (f_{0-if}= 451kHz, and f_{1-if}= 459kHz). The output of the multiplier comprises cos(2πf) * cos(2πf + θ), or (1/2)cos(θ) + (1/2)cos(4πf + θ). A low pass filter (LPF 164) removes the AC component of this product ((1/2)cos(4πf + θ)), and allows only the DC component ((1/2)cos(θ)) to pass as analog signal 165. Because θ produced by the phase shift block 162 is different at f_{0-if} and f_{1-if}, the data becomes apparent at this point, although it may be substantially noisy.

The limiting amplifier 158 and multiplier 160 can comprise portions of the same demodulator integrated circuit, such as Part No. SA608DK, manufactured by NXP Semiconductors N.V.

The analog signal 165 is provided to an Analog-to-Digital converter (A/D) block 172, which can comprise a discrete block or an A/D input of a microcontroller 170 of the external controller 12 as shown. The signal 165 is sampled at an appropriate rate, and the resulting digitized values of the amplitude of the signal 165 at different points in time are stored in memory 174. Once stored, a digital filter 176, operating as software in the microcontroller 170, can operate on the stored data to remove noise and recover the data as a digital bit stream 177. The particulars of filter 176 are not important, and are not further discussed.

While the receiver and demodulation circuitry 150 of the prior art external controller 12 of Figure 3 functions well, the inventors see room for improvement. First, circuitry 150 is relatively expensive, as it uses relatively expensive components, such as the demodulator IC and the ceramic band pass filter(s). There is also unnecessary complexity in up-shifting the frequency from the natural center at which it is transmitted (f_{c}=125 kHz) to a higher intermediate frequency (f_{c-if}=455 kHz) simply to accommodate the use of hardware designed to operate at this conventional frequency. Further, circuitry 150 has reliability and manufacturing concerns. The ceramic band pass filter(s) are fragile and can break, which is of particular concern in an external controller 12 that may from time to time be dropped by the patient. The quad coil 163 in the phase shift block 162 is also difficult to work with, as it requires special handling in manufacturing, and must be tuned by hand to ensure that it provides the proper 90-degree shift at the center frequency f_{c-if}=455 kHz.

Given these shortcomings, the art of implantable medical devices would benefit from improved receiver and demodulation circuitry for an external controller, and this disclosure presents solutions. US-A-2005/0131494 describes a system for communicating with an implantable stimulator and including a coil configured to transmit a signal modulated with on-off keying (OOK) modulation to transmit control data.

### BRIEF DESCRIPTION OF THE DRAWINGS

Figure 1 shows an Implantable Pulse Generator (IPG) in accordance with the prior art.
Figures 2A and 2B show an external controller for communicating with an IPG in accordance with the prior art.
Figure 3 shows receiver and demodulation circuitry useable in the external controller of the prior art.
Figure 4 shows improved receiver and demodulation circuitry useable in an external controller in accordance with an embodiment of the invention.
Figure 5 shows problems with the use of a single band pass filter in receiver and demodulation circuitry for an external controller.
Figure 6 shows the frequency responses for the two band pass filters used in accordance with an embodiment of the invention.
Figure 7 shows received data being demodulated using a clock of the microcontroller in accordance with an embodiment of the invention.
Figure 8 shows further details of the demodulation circuitry in accordance with an embodiment of the invention.
Figure 9 shows operation of the demodulation circuitry of Figure 8 in accordance with an embodiment of the invention.

### DETAILED DESCRIPTION

Improved receiver and demodulation circuitry for an external controller that receives a band of frequencies (e.g., FSK) from an implantable medical device is disclosed. The improved circuitry comprises two relatively sharp, narrow-band-width (high Q) band pass filters (BFPs) connected in series. Each BFP is tuned to a different center frequency, such that these center frequencies are outside the band of interest (e.g., f₀ = 121 kHz and f₁ = 129 kHz). When connected in series, the resulting frequency response is suitably wide to receive the band without attenuation, but sharply rejects noise outside of the band. The received frequencies are not up-shifted to an intermediate frequency, which simplifies receiver design. Moreover, the BPFs are formed of standard, low-cost components, such as resistors, capacitors, and operational amplifiers.

The resulting filtered AC signal is input to a comparator to produce a square wave of the filtered signal. This square wave maintains the frequencies of the received signal, yet is suitable for input to s digital input of a microcontroller in the external controller without conversion.

Demodulation of the square wave is accomplished exclusively in software in the microcontroller, and does not require a multiplier or a quad coil, further simplifying the design. Demodulation involves assessing in the microcontroller the time between transitions in the square wave, and comparing those times to expected transition times for the logic states in the data ('0' or '1'). These transition times can be determined and compared using the known timing of the microcontroller's clock as a reference. The results of these comparisons are stored and filtered to remove noise and to recover the data transmitted by the implantable medical device.

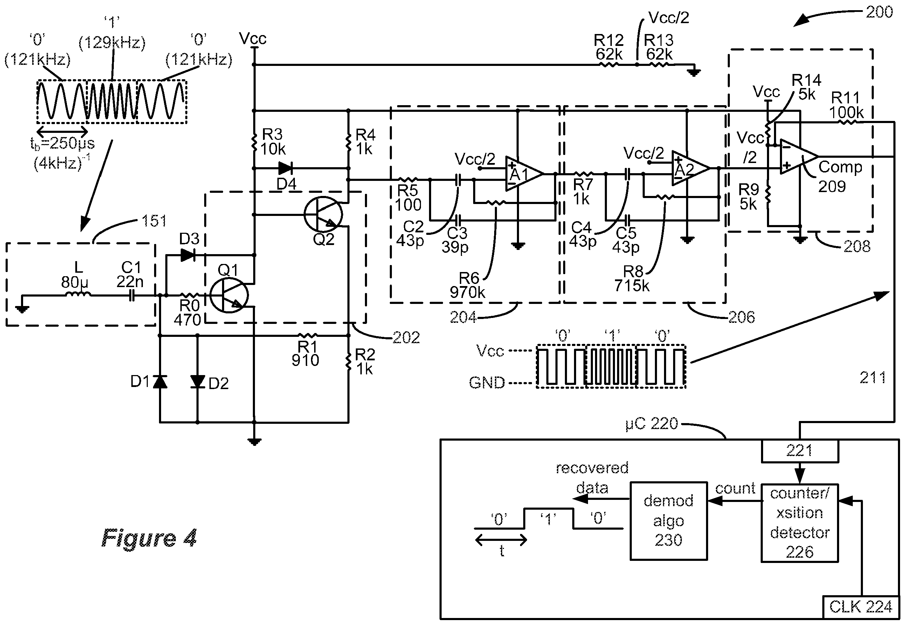

Figure 4 shows an embodiment of improved receiver and demodulation circuitry 200 for an external controller 12. The improved circuitry 200, like the prior art circuitry 151, comprises a tank circuit 151 with a telemetry coil 73 and a tank capacitor C, which can be connected in series as shown or in parallel (not shown). As before, the values for these components are chosen to generally resonate at the center frequency f_{c}=125 kHz of the FSK data expected from the IPG 100. Example values for the various resistances, capacitances, and inductances in circuitry 200 are shown in Figure 4.

The small AC signal from the coil 73 is provided to an amplifier 202, which is shown as a cascaded arrangement of bipolar transistors Q1 and Q2. See, e.g., http://en.wikipedia.org/wiki/Cascade_amplifier. As one skilled in the art will recognize, each bipolar transistor amplifies the signal coming into its base. Different numbers of transistors could also be used, such as a single transistor, three cascaded transistors, etc. Diodes D1-D4 provide overvoltage protection and are not strictly necessary. The particulars of amplifier 202 are not important, and amplifier 202 could be made in other ways, although the disclosed circuit is preferred because of its simplicity, reliability, and the low cost of its components. Other types of amplifier circuits could also be used.

The output of the amplifier 202 is then band pass filtered, although in the improved circuit 200, the frequency of the received signal is not up-shifted to an intermediate frequency; this reduces complexity, because a mixer 154 and a reference waveform (330 kHz) (Fig. 3) are not necessary.

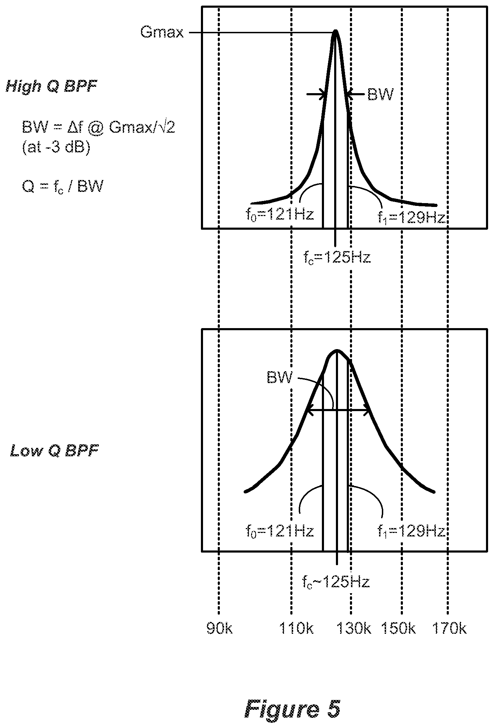

An ideal band pass filter would pass the frequencies of interest (f₀ = 121 kHz; f₁ = 129 kHz), while completely rejecting frequencies outside of this band. A band pass filter would also preferably comprise an active filter using standard, inexpensive components, such as resistors, capacitors, and operational amplifiers (op amps). However, the inventors consider it difficult to suitably filter the received FSK signals using a single traditional band pass filter. Figure 5 shows frequency responses for a single bass pass filter having a relatively high Quality Factor (Q) and a relatively low Q value. The high Q filter, as one skilled in the art understands, has relatively steep sides, meaning that it will reject out-of band frequencies more easily. However, a high Q filter necessarily has a smaller bandwidth (BW), as governed by the inverse relationship between them reflected in the formulas in Figure 5. If Q is too high and the bandwidth is too small, the FSK frequencies of interest f₀ and f₁ will be overly attenuated by the filter, which is not preferable. The low Q filter, by contrast, has relatively sloped sides, and a larger bandwidth. The FSK frequencies are thus not as attenuated by the filter, but the filter will pass a greater amount of out-of-band signals (noise), which is also not preferable. Moreover, in either case, frequencies between f₀ and f₁, such as f_{c}, are passed with higher gains, which is unnecessary, and which in effect tends to amplify noise within the band.

The inventors' solution uses two band pass filters 204 and 206 in series as shown in Figure 4, each with a relatively high Q value. In particular, a preferred design for each BPF 204 and 206 is an Infinite Gain Multiple Feedback Active filter, as described in http://www.electronics-tutorials.ws/filter/filter_7.html, which is submitted herewith. Each filter 204 and 206 is configured similarly, with each having an input resistor (R5 and R7), an input capacitor (C2 and C4), a feedback resistor (R6 and R8), a feedback capacitor (C3 and C5), and an op amp (A1 and A2).

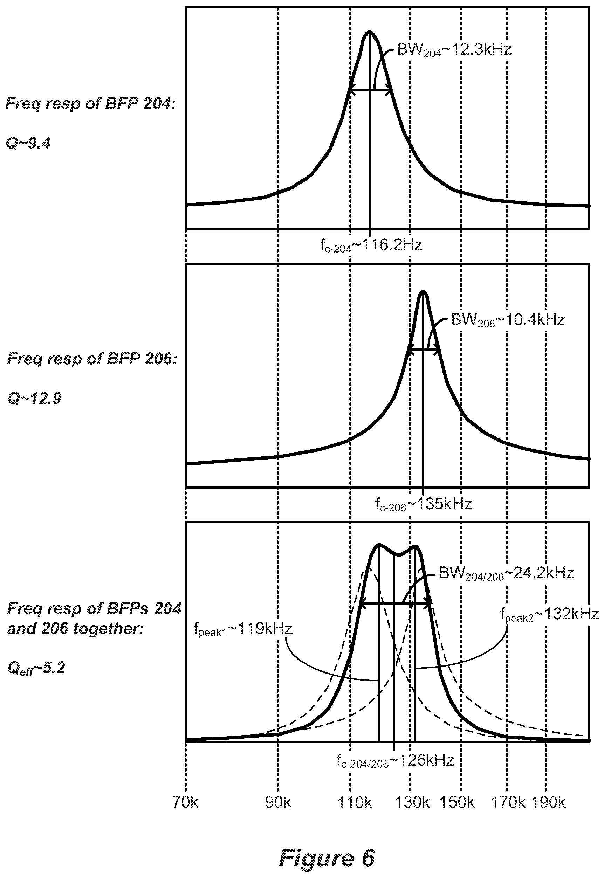

Each of the BPFs 204 and 206 are tuned to a different center frequency (f_{c-204}; f_{c-206}), as shown in Figure 6, which illustrates simulated frequency responses for the improved circuit of Figure 5. As shown at the top, the first BPF 204 is tuned to a center frequency of approximately f_{c-204} = 116.2 kHz, slightly below f₀ = 121 kHz. As shown in the middle, the second BPF 206 is tuned to a center frequency of approximately f_{c-206} = 135 kHz, slightly above f₁=129 kHz. The bandwidths for each BPF 204 and 206 are relatively small (12.3 kHz and 10.4 kHz respectively), and thus the Q values are thus relatively high (9.4 and 12.9 respectively).

The bottom figure shows the simulated frequency response for both BPFs 204 and 206 connected in series, with the frequencies responses for each of the individual BFPs 204 and 206 overlaid for comparison. As can be seen, the combined frequency response comprises two peaks, roughly centered at fₚₑₐₖ₁ = 119 kHz and fₚₑₐₖ₂ = 132 kHz, values which are within the range of the centers of each of the BPFs considered individually (i.e., f_{c-204} = 116.2 kHz and f_{c-206} = 135 kHz), but which encompass the FSK frequencies of interest (i.e., f₀ = 121 kHz and f₁ = 129 kHz). As such, the combined BFPs 204 and 206 will suitably pass the desired frequencies, and thus acts as a relatively low Q filter in this respect: if one considers 126 kHz as the center frequency of the combined BFPs 204 and 206, the effective Q value, Qeff, can be estimated as 5.2. At the same time, frequencies are attenuated relatively sharply outside of the passed band, and the overlays in the bottom figure show that the frequency response of the combined filters falls off at essentially the same rate as do each of the BPFs 204 and 206 individually. In this respect, the combined BFPs 204 and 206 act as a high Q filter with steep walls.

In summary, the combined effect of the BFPs 204 and 206 is a filter with sharp walls for good noise rejection, and a suitable bandwidth to pass the FSK frequencies of interest. Moreover, such performance is achieved using inexpensive components, which, unlike the ceramic BPFs describes earlier, are not prone to breaking. Moreover, frequencies between the FSK frequencies of interest (e.g., from 121 kHz to 129 kHz) are not accentuated by the BPFs 204 and 206, and in fact may be slightly attenuated, which is beneficial compared to the use of a single BFP alone, as discussed earlier with reference to Figure 5.

As explained in the incorporated materials, the center frequency, bandwidth, and Q values for each BPF 204 and 206 can be tailored by adjusting the various values for the resistances and the capacitances in each stage, and equations for doing so are provided. However, while such equations will generally help one skilled in the art to tailor the frequency responses of the individual BFPs 204 and 206, such skilled persons will also recognize that determining suitable values for the various resistors and capacitors may require routine simulation or experimentation. This is especially true when one considers the various parasitic resistances and capacitances at the input and output of each stage 204 and 206, and the input and output resistance of the op amps A1 and A2.

Referring again to Figure 4, after band pass filtering, the AC signal is provided to a comparator stage 208 where it is digitized. The AC signal provided to the non-inverting input of comparator 209 in the comparator stage 208 ranges around Vcc/2 (i.e., one-half of the power supply voltage Vcc/2) by virtue of the non-inverting input to the op amp A2 in BFP 206. The comparator 209 is likewise reference at its inverting input to Vcc/2, and so comparator 209 outputs a square wave 211 between Vcc and ground and (ideally) with a frequency of either of f₀ or f₁-the FSK frequencies of interest requiring demodulation. Of course, noise passed by BFPs 204 and 206 will also be passed by comparator 209, and therefore the square wave 211 will not necessarily transition only in accordance with f₀ and f₁.

Resistor R11 in the comparator stage 208 provides hysteresis to avoid glitches in the comparator 209's output as it transitions between states. This hysteresis pulls the inverting input to the comparator 209 slightly lower when the output goes low, and slightly higher when the output goes high. Such hysteresis also provides a squelch function by preventing small signals from triggering the comparator 209. Squelching is not strictly required, but if provided, resistor R11 Should not be too small or squelching will be too great and only large signals will be received, thus decreasing the receiver's sensitivity. As shown, R11 is connected to Vcc/2 by virtue of the voltage divider formed by resistors R14 and R9. This is potentially problematic, because noise on Vcc could affect the output of the comparator 209. Therefore, the Vcc/2 reference provided at the inverting input is preferably decoupled from Vcc and the other Vcc/2 references provided to the amplifiers A1 and A2. This allows beneficial hysteresis and squelching to occur in the comparator stage 208 without being adversely affecting by other circuits.

In the prior art discussed previously with respect to Figure 3, the data input to the microcontroller 170 comprised analog amplitude data, which had to be digitized (172) before it could be filtered (176) to recover the transmitted data. By contrast, in the improved circuitry 200, the square wave 211 received at the microcontroller 220 is digital, as it varies between Vcc and ground, and is not indicative of the amplitude of the received signal. As such, the received signal need not be input to A/D circuitry, or to A/D inputs of the microcontroller 170, but instead can be provided to digital inputs 221 of the microcontroller 170, which may comprise the data bus by which the microcontroller 220 normally receives data. This marks yet another improvement over the prior art, as digital data is easier to handle and subsequently process in the microcontroller 220. A/D conversion, by contrast, is computationally intensive.

The square wave 211 still needs to be demodulated, and such demodulation occurs exclusively in the microcontroller 220 by analyzing the transitions in the square wave 211. Unlike the prior art discussed earlier, demodulation in improved circuitry 200 does not require a multiplier 160 and phase shift block 162 (Fig. 3). This simplifies the external controller's design, and reduces cost and manufacturing complexity, in particular because improved circuitry 200 contains no quad coil (163; Fig. 3) that must be tuned by hand.

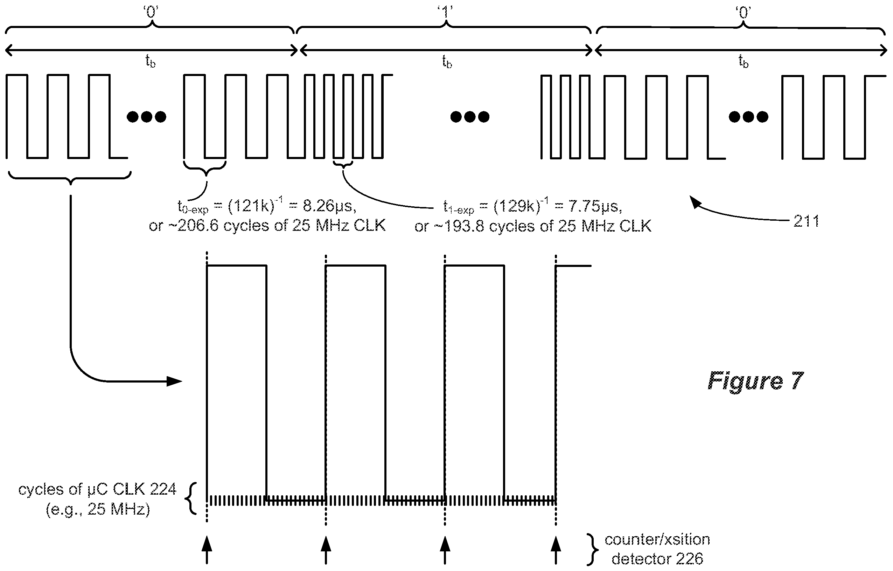

As shown in Figure 4, the square wave 211 is sent to a counter/transition detector block 226 whose output is provided to a demodulation algorithm 230, both of which preferably operate as software programmed into the microcontroller 220. The basic operation of block 226 is illustrated in Figure 7. The goal of counter/transition detector 226 is to identify rising edge transitions in the square wave 211, and to count the number of microcontroller clock cycles (CLK 224) that have occurred between such transitions. In effect, this strategy measures the time between rising edge transitions using the known timing of the CLK as a reference. Figure 7 shows the expected transition timings for a '0' bit (t₀₋ₑₓₚ) and a '1's bit (t₁₋ₑₓₚ), which assuming the use of a 25 MHz clock, comprise approximately 206.6 clock cycles and 193.8 clock cycles respectively. Falling edges of the square wave 211, or both rising and falling edges, could also be assessed, but this is not shown in subsequent examples for simplicity.

Demodulation occurs in the microcontroller 220 by counting these clock cycles, and comparing them to expected values to recover the data. These details are explained subsequently, but a simple example illustrates the principle. If for example the block 226 sees that the last five transitions comprised 207, 204, 206, 206, and 205 clock cycles, it may start to understand that a '0' bit has been received, and that subsequent transitions would yield similar numbers of clock cycles for a bit duration of t_{b}. Likewise, if block 226 sees that the last five transitions comprised 192, 193, 196, 194, and 193 clock cycles, it may start to understand that a '1' bit has been is being received, which again will continue for t_{b}.

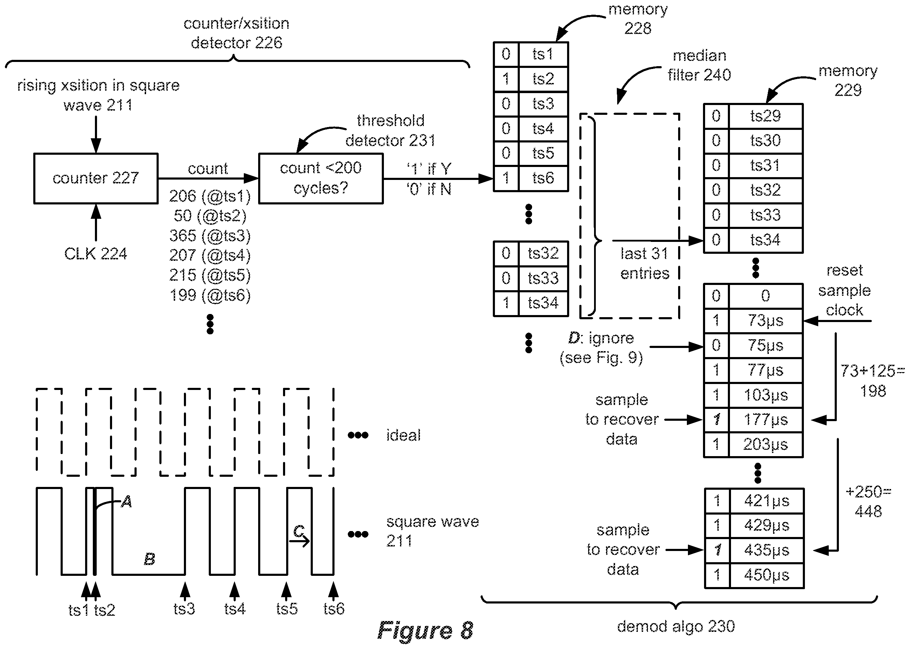

Of course, this simple example assumes no noise in the square wave 211. The bottom of Figure 8 shows an example square wave 211 having different types of noise, such as a spike (point A), a missing transition (point B), and a transitions shifted in time (point C). Such noise can arise due to any number of factors.

Figure 8 further illustrates the counter/transition detector 226 and the demodulation algorithm 230, and shows the ways in which noise is handled by the improved circuitry 200. Because implemented in software in the microcontroller 220, one skilled will understand that the blocks shown Figure 8 may comprise logical structures, which could be implemented in any numbers of ways.

Working with the noisy square wave 211, a counter 227 counts the number of clock cycles of CLK 224 at each rising transitions of the square wave 211. Assuming that the square wave 211 encodes a '0' bit, such transitions should occur approximately every t₀₋ₑₓₚ = 206 clock cycles, such as occurs at time stamp 1 (ts1). A threshold detector 231 compares this count to a threshold between t₀₋ₑₓₚ and t₁₋ₑₓₚ, such as 200 for example. If the count is below this threshold, the threshold detector outputs a '1'; if above, it outputs a '0'. These values are stored in a memory 228 along with its time stamp, which can comprise any timing reference typically provided in the microcontroller 220. Thus, the various counts (206, 50, 365, 207, 215, 199) have been reduced to single bits (0, 1, 0, 0, 0, 1) and stored in the memory 228 with their time stamps as shown.

Thereafter, a filter, such as a median filter 240, assesses some number of the latest entries in the memory 228 to determine which logic state is predominating. In one example, the median filter 240 can assess the last 31 entries in the memory 228, which roughly corresponds to the expected number of transitions in the square wave 211 assuming no noise (i.e., f_{c} = 125 kHz / 4 kbit/s). Noisier square waves 211 may have higher numbers of transitions per bit, in which case the median filter 240 may not assess all transitions in the bit, but this is acceptable. Alternatively, the median filter 240, instead of assessing a fixed number of transitions stored in the memory 228, could assess all transitions occurring over a set time period, such as 250 microseconds, which corresponds to the bit duration, t_{b}. Logging of time stamps in the memory 228 would allow the median filter 240 to operate in this way. The median filter 240 can thus be implemented in different ways, and the filter shown is merely one example.

The median filter 240 outputs the predominant logic state in the latest entries in memory 228 (i.e., the logic state with 16 or more entries) to another memory 229, along with the time stamp of the latest transitions the median filter considered. As explained subsequently, the time stamps will be used to sample the memory 229 to recover the data. Although the time stamps in memory 228 are shown as re-recorded in memory 229, this is merely for simplicity and need not actually occur, as the memory 229 can instead make reference to the time stamps in memory 228.

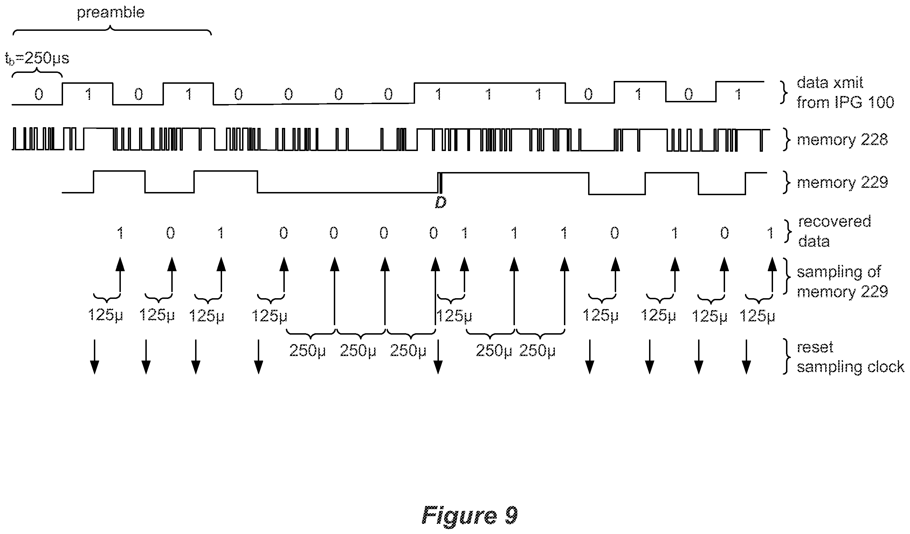

As just mentioned, the output of the median filter 240, i.e., memory 229, is sampled to recover the data, and this is shown in Figure 9. Generally speaking, the goal is to sample the memory 229 in the middle of the bits, which timing is determined using by discerning where transitions in the received data bits have occurred, and knowledge of the bit duration, t_{b}.

An example bit stream as transmitted from the IPG 100 is shown at the top of Figure 9. An alternating preamble (0101) can precede the transition of actual data, which is useful to provide known transitioning bit data to synchronize the sampling clock used to sample memory 229, as discussed further below. Also shown are the latest contents of memories 228 and 229 as a function of time. As can been seen, the data in memory 228 is rather noisy, but operation of the median filter 240 has operated to remove much of that noise in memory 229.

The values stored in memory 229 are monitored to determine when a bit transition has taken place. Such transitions reset a sampling clock, to which 125 microseconds ((1/2)t_{b}) are added for sampling the memory 229. This is shown in the example of Figure 8. Notice that the memory 229 transitioned to a '1' at a time stamp of 73 µs. At this point, the sampling clock is reset to 73 µs and 125 µs is added to this value (198 µs). This value is compared to the time stamps stored in the memory 229, and it is seen that time stamp 177 µs is the latest time stamp preceding 198 µs. The bit associated with this time stamp ('1') is thus sampled as the recovered data. Alternatively, the bit associated with the time stamp nearest to 198 µs (i.e., 203 µs) could also be chosen for sampling.

Should there be no transition in the data, the sampling clock is not reset, and instead another 250 µs is added to it, which should correspond to the center of the next (non-transitioning) bit. This new value (448 µs) is then used to sample the memory 229, which as illustrated corresponds to the entry with the time stamp of 435 µs (again, 450 µs could also have been chosen as the value closest to 448 µs). Should a transition thereafter be apparent in the data in memory 229, the sampling clock would again be reset. Resetting the sampling clock on transitions in the data is preferred in case the time basis of the data drifts.

Sampling in the middle of the bits is preferred, as operation of the median filter 240 may not be perfect, and "glitches" can occur (point D, Fig. 9), particularly at the transitions between bits. Such glitches may simply be ignored, and not used to reset the sampling clock. For example, transitions occurring some time after a sampling clock reset (10 µs, 125 µs, or 230 µs, which is just short of t_{b}) may be ignored and not used to reset the sampling clock.

The disclosed technique can also operate to receive, filter, and demodulate more than two FSK frequencies, i.e., Multi-Frequency shift keying in which N number of symbols are transmitted at N different frequencies. For example, symbols '00,' '01,' '10,' and '11' could be represented by transmitted frequencies f₁, f₂, f₃, ad f₄, thus allowing each frequency to transmit two bits of data. Each of these frequencies would be within the band pass of the BFPs 204 and 206, and the counter/transition detector 226 would be modified to compare the number of counts between transitions to three thresholds between the four expected numbers of counts for each of the frequencies.

"Microcontroller" as used herein should be broadly construed as including all sorts of logic circuits capable of performing the various functions describe herein, including microprocessors, digital signal processors, and the like.

The present invention is set out in the claims that follow. The embodiments, aspects or examples according to the present description that do not fall within the scope of said claims are provided for illustrative purposes only and do not form part of the present invention.

## Claims

1. An external controller (12) for receiving wireless data from an implantable medical device (100), comprising:
an antenna configured to generate an AC signal in response to wireless data from the implantable medical device (100), wherein the AC signal comprises at least two data frequencies in the wireless data each indicative of a data state;
a receiver circuitry configured to receive the AC signal, wherein the receiver circuitry comprises first (204) and second (206) band pass filters, wherein the first band pass filter (204) receives an output of an amplifier (152), and wherein the second band pass filter (206) receives an output of the first band pass filter (204), wherein the first band pass filter (204) is centered at a first frequency, and wherein the second band pass filter (206) is centered at a second frequency, the receiver circuitry further comprising a comparator (209) for receiving an output of the second band pass filter (206) and configured to produce a square wave comprised of the at least two data frequencies; and
a microcontroller (170) configured to receive the square wave, wherein the microcontroller (170) is configured to demodulate the square wave to recover the data states by determining timings between transitions in the square wave.

2. The external controller (12) of claim 1, wherein the antenna comprises a tank circuit (151) comprising a coil (73), wherein the antenna preferably comprises the amplifier (152) coupled to the tank circuit (151).

3. The external controller (12) of claim 1, wherein the at least two data frequencies are between the first and second frequencies.

4. The external controller (12) of claim 1, wherein the square wave is input to a digital input of the microcontroller (170).

5. The external controller (12) of claim 1, wherein the microcontroller (170) is configured to determine timings between transitions in the square wave by counting a number of clock cycles between transitions in the square wave.

6. The external controller (12) of claim 5, wherein the clock cycles come from a clock signal internal to the microcontroller (170).

7. The external controller (12) of claim 1, wherein the microcontroller (170) is configured to recover the data states by comparing the timings between transitions in the square wave to expected timings between transitions for each of the data frequencies.

8. The external controller (12) of claim 1, wherein the microcontroller (170) is configured to recover the data states by comparing the timings between transitions in the square wave to a threshold value, wherein the threshold value is between expected timings between transitions for each of the data frequencies.

9. The external controller (12) of claim 1, wherein the microcontroller (170) further comprises a memory (174), wherein data indicative of the timing between transitions in the square wave are stored in the memory (174).

10. The external controller (12) of claim 9, wherein the microcontroller (170) comprises a median filter (176), wherein the median filter (176) assesses some number of the most recent entries in the memory (174).

11. The external controller (12) of claim 10, wherein an output of the median filter (176) is sampled to recover the data.

## Patentansprüche

1. Externe Steuerung (12) zum Empfangen drahtloser Daten von einem implantierbaren Medizinprodukt (100), die aufweist:
eine Antenne, die so konfiguriert ist, dass sie ein Wechselstromsignal als Reaktion auf drahtlose Daten von dem implantierbaren Medizinprodukt (100) erzeugt, wobei das Wechselstromsignal mindestens zwei Datenfrequenzen in den drahtlosen Daten aufweist, die jeweils einen Datenzustand angeben;
einen Empfängerschaltungsaufbau, der so konfiguriert ist, dass er das Wechselstromsignal empfängt, wobei der Empfängerschaltungsaufbau ein erstes (204) und ein zweites (206) Bandpassfilter aufweist, wobei das erste Bandpassfilter (204) eine Ausgabe eines Verstärkers (152) empfängt und wobei das zweite Bandpassfilter (206) eine Ausgabe des ersten Bandpassfilters (204) empfängt, wobei das erste Bandpassfilter (204) auf einer ersten Frequenz zentriert ist und wobei das zweite Bandpassfilter (206) auf einer zweiten Frequenz zentriert ist, wobei der Empfängerschaltungsaufbau ferner einen Komparator (209) zum Empfangen einer Ausgabe des zweiten Bandpassfilters (206) aufweist, der so konfiguriert ist, dass er eine aus den mindestens zwei Datenfrequenzen bestehende Rechteckwelle erzeugt; und
einen Mikrocontroller (170), der so konfiguriert ist, dass er die Rechteckwelle empfängt, wobei der Mikrocontroller (170) so konfiguriert ist, dass er die Rechteckwelle demoduliert, um die Datenzustände durch Bestimmen von Zeiten zwischen Übergängen in der Rechteckwelle rückzugewinnen.

2. Externe Steuerung (12) nach Anspruch 1, wobei die Antenne einen Tankkreis (151) mit einer Spule (73) aufweist, wobei die Antenne vorzugsweise den Verstärker (152) aufweist, der mit dem Tankkreis (151) gekoppelt ist.

3. Externe Steuerung (12) nach Anspruch 1, wobei die mindestens zwei Datenfrequenzen zwischen der ersten und zweiten Frequenz liegen.

4. Externe Steuerung (12) nach Anspruch 1, wobei die Rechteckwelle zu einem digitalen Eingang des Mikrocontrollers (170) eingegeben wird.

5. Externe Steuerung (12) nach Anspruch 1, wobei der Mikrocontroller (170) so konfiguriert ist, dass er Zeiten zwischen Übergängen in der Rechteckwelle durch Zählen einer Anzahl von Taktzyklen zwischen Übergängen in der Rechteckwelle bestimmt.

6. Externe Steuerung (12) nach Anspruch 5, wobei die Taktzyklen von einem Taktsignal innerhalb des Mikrocontrollers (170) stammen.

7. Externe Steuerung (12) nach Anspruch 1, wobei der Mikrocontroller (170) so konfiguriert ist, dass er die Datenzustände durch Vergleichen der Zeiten zwischen Übergängen in der Rechteckwelle mit erwarteten Zeiten zwischen Übergängen für jede der Datenfrequenzen rückgewinnt.

8. Externe Steuerung (12) nach Anspruch 1, wobei der Mikrocontroller (170) so konfiguriert ist, dass er die Datenzustände durch Vergleichen der Zeiten zwischen Übergängen in der Rechteckwelle mit einem Schwellwert rückgewinnt, wobei der Schwellwert zwischen erwarteten Zeiten zwischen Übergängen für jede der Datenfrequenzen liegt.

9. Externe Steuerung (12) nach Anspruch 1, wobei der Mikrocontroller (170) ferner einen Speicher (174) aufweist, wobei Daten als Angabe der Zeit zwischen Übergängen in der Rechteckwelle im Speicher (174) gespeichert werden.

10. Externe Steuerung (12) nach Anspruch 9, wobei der Mikrocontroller (170) ein Medianfilter (176) aufweist, wobei das Medianfilter (176) eine gewisse Anzahl der jüngsten Eintragungen im Speicher (174) beurteilt.

11. Externe Steuerung (12) nach Anspruch 10, wobei eine Ausgabe des Medianfilters (176) abgetastet wird, um die Daten rückzugewinnen.

## Revendications

1. Dispositif de commande externe (12) destiné à recevoir des données sans fil en provenance d'un dispositif médical implantable (100), comportant :
une antenne configurée de manière à générer un signal alternatif en réponse à des données sans fil provenant du dispositif médical implantable (100), dans lequel le signal alternatif comporte au moins deux fréquences de données dans les données sans fil, chaque fréquence indiquant un état de données ;
un montage de circuits récepteurs configuré de manière à recevoir le signal alternatif, dans lequel le montage de circuits récepteurs comporte des premier (204) et second (206) filtres passe-bande, dans lequel le premier filtre passe-bande (204) reçoit une sortie d'un amplificateur (152), et dans lequel le second filtre passe-bande (206) reçoit une sortie du premier filtre passe-bande (204), dans lequel le premier filtre passe-bande (204) est centré à une première fréquence, et dans lequel le second filtre passe-bande (206) est centré à une seconde fréquence, le montage de circuits récepteurs comportant en outre un comparateur (209) destiné à recevoir une sortie du second filtre passe-bande (206) et configuré de manière à produire une onde carrée constituée d'au moins deux fréquences de données ; et
un microcontrôleur (170) configuré de manière à recevoir l'onde carrée, dans lequel le microcontrôleur (170) est configuré de manière à démoduler l'onde carrée en vue de récupérer les états de données en déterminant des temporisations entre des transitions dans l'onde carrée.

2. Dispositif de commande externe (12) selon la revendication 1, dans lequel l'antenne comporte un circuit oscillant final (151) comportant une bobine (73), dans lequel l'antenne comporte de préférence l'amplificateur (152) couplé au circuit oscillant final (151).

3. Dispositif de commande externe (12) selon la revendication 1, dans lequel lesdites au moins deux fréquences de données se situent entre les première et seconde fréquences.

4. Dispositif de commande externe (12) selon la revendication 1, dans lequel l'onde carrée est entrée dans une entrée numérique du microcontrôleur (170).

5. Dispositif de commande externe (12) selon la revendication 1, dans lequel le microcontrôleur (170) est configuré de manière à déterminer des temporisations entre des transitions dans l'onde carrée, en comptant un nombre de cycles d'horloge entre des transitions dans l'onde carrée.

6. Dispositif de commande externe (12) selon la revendication 5, dans lequel les cycles d'horloge proviennent d'un signal d'horloge interne au microcontrôleur (170).

7. Dispositif de commande externe (12) selon la revendication 1, dans lequel le microcontrôleur (170) est configuré de manière à récupérer les états de données en comparant les temporisations entre des transitions dans l'onde carrée à des temporisations attendues entre des transitions pour chacune des fréquences de données.

8. Dispositif de commande externe (12) selon la revendication 1, dans lequel le microcontrôleur (170) est configuré de manière à récupérer les états de données en comparant les temporisations entre des transitions dans l'onde carrée à une valeur de seuil, dans lequel la valeur de seuil se situe entre des temporisations attendues entre des transitions pour chacune des fréquences de données.

9. Dispositif de commande externe (12) selon la revendication 1, dans lequel le microcontrôleur (170) comporte en outre une mémoire (174), dans lequel des données indicatives de la temporisation entre des transitions dans l'onde carrée sont stockées dans la mémoire (174).

10. Dispositif de commande externe (12) selon la revendication 9, dans lequel le microcontrôleur (170) comporte un filtre médian (176), dans lequel le filtre médian (176) évalue un nombre des entrées les plus récentes dans la mémoire (174).

11. Dispositif de commande externe (12) selon la revendication 10, dans lequel une sortie du filtre médian (176) est échantillonnée en vue de récupérer les données.
